(19) Europäisches Patentamt
European Patent Office
Office européen des brevets

(11) Veröffentlichungsnummer : **0 043 548**
**B1**

(12) # EUROPÄISCHE PATENTSCHRIFT

(45) Veröffentlichungstag der Patentschrift : **25.07.84**

(51) Int. Cl.³ : **C 07 D307/52**, C 07 D295/14, A 61 K 31/635, A 61 K 31/40, A 61 K 31/275, A 61 K 31/135

(21) Anmeldenummer : **81105087.1**

(22) Anmeldetag : **01.07.81**

(54) **Salz aus Furosemid oder Piretanid als Säurekomponente und Penbutolol als Basenkomponente sowie Arzneimittel, das beide Komponenten enthält.**

(30) Priorität : **04.07.80 DE 3025367**

(43) Veröffentlichungstag der Anmeldung : **13.01.82 Patentblatt 82/02**

(45) Bekanntmachung des Hinweises auf die Patenterteilung : **25.07.84 Patentblatt 84/30**

(84) Benannte Vertragsstaaten : **AT BE CH DE FR GB IT LI NL SE**

(56) Entgegenhaltungen :
**AT-B- 356 095**
**DE-A- 2 227 423**
**DE-A- 2 638 716**
**Die Akte enthält technische Angaben, die nach dem Eingang der Anmeldung eingereicht wurden und die nicht in dieser Patentschrift enthalten sind.**

(73) Patentinhaber : **HOECHST AKTIENGESELLSCHAFT**
**Postfach 80 03 20**
**D-6230 Frankfurt am Main 80 (DE)**

(72) Erfinder : **Muschaweck, Roman, Dr.**
**Heimchenweg 39**
**D-6230 Frankfurt am Main 80 (DE)**
Erfinder : **Fülberth, Werner, Dr.**
**Theodor-Storm-Strasse 11**
**D-6233 Kelkheim Taunus (DE)**
Erfinder : **Sickmüller, Alfred, Dr.**
**Schweinfurter Weg 72**
**D-6000 Frankfurt am Main 70 (DE)**



Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Herz-Kreislauf-Erkrankungen stellen einen erheblichen Risikofaktor für die Lebenserwartung dar. Insbesondere die Behandlung des Hochdrucks ist deshalb eine unabdingbare Forderung.

Es ist bekannt, daß der Bluthochdruck sowohl mit Diuretika als auch mit β-Blockern behandelt werden kann.

Bei der Behandlung des Hochdrucks mit Diuretika werden sowohl Verbindungen des Typs eines « Low-ceiling » als auch Verbindungen des Typs eines « high-ceiling »-Diuretikums angewandt. Unter einem high-ceiling-Diuretikum wird ein solches mit raschem Wirkungseintritt, starker Wirkung und baldigem Abklingen der Wirkung verstanden. Ein low-ceiling-Diuretikum ist ein solches mit langsamem Wirkungseintritt und langanhaltender Wirkung. Die Beeinflussung des Hochdrucks durch ein Diuretikum kommt im wesentlichen durch die erhöhte Ausscheidung von Wasser und Natrium-Ionen zustande, wodurch eine verminderte Volumenbelastung des Kreislaufs bewirkt wird.

Im Gegensatz dazu kommt es bei Einsatz eines β-Blockers, oftmals erst nach längerer Behandlungsdauer, zu einer Reduktion des Blutdrucks, die nach einem anderen, noch nicht geklärten Mechanismus abläuft.

Es ist eine Reihe von Kombinationen von Diuretika mit β-Blockern für die Behandlung des Hochdrucks bekannt, bei denen unterschiedliche Wechselwirkungen zwischen den Kombinationspartnen beobachtet werden. In einigen Fällen setzt sich die Wirkung additiv aus der der beiden Einzelkomponenten zusammen, in anderen Fällen kommt es zu mehr oder weniger erheblichen Wirkungsbeeinträchtigungen bis hin zur Unwirksamkeit.

Beispiele bekannter Kombinationen sind die folgenden :

Pindolol und Hydrochlorothiazid (Med. J. Aust. 1/18, 650-653 (1976)),
Timolol und Bendrofluazid (Clin. Trials J. 14/5, 173-180 (1977)),
Timolol-Maleat und Bendrofluazid (J. int. Med. Res. 5/2, 114-119 (1977)),
Pindolol und Hydrotrichlorothiazid (Z. Kardiol. 66/9, 508-510 (1977)),
Atenolol und Chlorthalidon (Brit. Med. J. 1/6053, 76-78 (1977)),
Oxprenolol und Chlorthalidon (Bol. Soc. Port. Kardiol. 15/3, 147-169 (1977)),
Propranolol und Chlorthalidon (Hell. Cardiol. Rev. 18/2, 162-166 (1977)),
Oxprenolol und Chlorthalidon (Invest. Med. Int. 5/2, 119-126 (1978)),
Oxprenolol und Chlorthalidon (Verh. dtsch. Ges. Inn. Med. 83, 325-327 (1977)).

Gegenstand der Erfindung ist eine neue Kombination eines Diuretikums und eines β-Blockers, die sich durch besondere Eigenschaften auszeichnet.

Die Kombination enthält als Diuretikum-Komponente Furosemid oder Piretanid und als β-Blocker-Komponente Penbutolol.

Die genannten Komponenten sind bekannte Verbindungen.

Furosemid ist die 4-Chlor-2-[(2-furanoylmethyl)-amino]-5-sulfamoyl-benzoesäure der Formel I

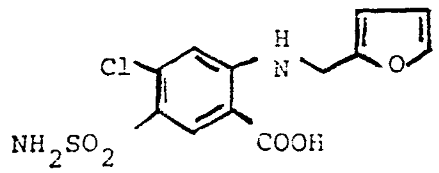

(I)

Piretanid ist die 4-Phenoxy-3-(1-pyrrolidinyl)-5-sulfamoylbenzoesäure, der die Formel II zukommt

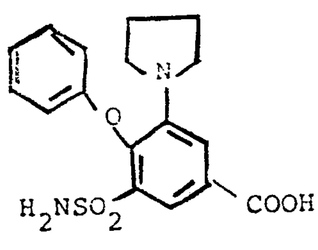

(II)

Penbutolol ist das (S)-(−)-(1-tert.-Butylamino)-3-(2-cyclopentylphenoxy)-2-propanol der Formel III

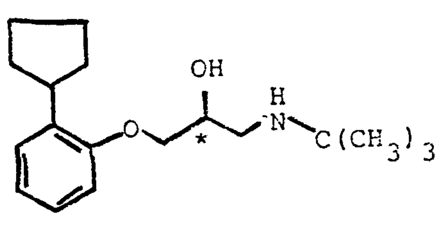

(III)

Die erfindungsgemäß verwendeten Diuretika sind in ihrer Eigenschaft als Carbonsäuren zur Salzbildung mit basischen Verbindungen befähigt. Demgegenüber besitzen erfindungsgemäß verwendeten β-Blocker basische Eigenschaften. Sie können demgemäß mit sauren Verbindungen Salze bilden und auch unter Salzbildung mit den Diuretika reagieren. Durch eine solche Reaktion entstehen neue Verbindungen, die Salzcharakter haben und die die pharmakologische Wirkung der sauren und der basischen Komponente in sich vereinigen.

Die Bildung von Salzen kann in einem gewissen Umfang bereits beim Zusammengeben und insbesondere beim Verpressen der trocknen Komponenten eintreten. Sie läßt sich gezielt erreichen, indem man Lösungen, insbesondere wäßrige oder alkoholische Lösungen, des Diuretikums einerseits und des β-Blockers andererseits zusammengibt und das Salz aus diesen Lösungen auskristallisiert.

Für die Verwendung als Pharmazeutikum eignen sich sowohl das Salz aus äquimolaren Mengen des Diuretikums und des β-Blockers als auch Mischungen beider Komponenten oder Mischungen des Salzes mit der einen und/oder anderen Komponente, wobei im Falle von Mischungen das Diuretikum und der β-Blocker nicht in äquimolaren Mengen vorzuliegen brauchen. Vielmehr kann dann das Verhältnis Diuretikum zu β-Blocker je nach der Lage des Krankheitsfalles variiert werden.

Gegenstand des Patentes ist demgemäß ein Salz aus Furosemid oder Piretanid als Säurekomponente und Penbutolol als Basenkomponente. Gegenstand des Patentes ist ferner ein Verfahren zur Herstellung eines solchen Salzes, das dadurch gekennzeichnet ist, daß man Furosemid oder Piretanid einerseits und Penbutolol andererseits, vorzugsweise in Form von Lösungen, zusammenfügt. Weiterhin ist Gegenstand des Patentes ein Arzneimittel, das mindestens zwei der Komponenten (A) Furosemid oder Piretanid, (B) Penbutolol und (C) ein Salz aus Furosemid oder Piretanid und Penbutolol enthält, sowie ein solches Arzneimittel zur Behandlung von Erkrankungen des Herz- und Kreislaufsystems.

Es ist bekannt, daß β-Blocker vielfach salurese- und diuresehemmende Eigenschaften besitzen. Eigene Untersuchungen zu diesem Effekt zeigten z. B. eine dosisabhängige Verminderung der Ausscheidungsgrößen sowohl von Urin als auch der Natrium-, Kalium- und Chlorionen unter der Wirkung von Pindolol. Dieser ausscheidungshemmende Effekt ist durch Diuretika nur schwer zu durchbrechen. So kann beispielsweise Hydrochlorothiazid auch in maximalen Dosierungen (1 bis 2 mg/kg) die diuresehemmende Wirkung von Pindolol kaum durchbrechen oder gar eine dem Präparat entsprechende Gesamtausscheidung hervorrufen. Gleiches gilt für die Wirkung von Furosemid bei gleichzeitiger Verabreichung von Pindolol.

Es gibt aber auch β-Blocker, die am normalen Versuchstier keine Hemmung der Diurese und Salurese zeigen. Hierzu gehört beispielsweise Penbutolol. Durch diese Verbindung wird bei oraler Gabe in Dosierungen von 5 bis 50 mg/kg die saluretische Wirksamkeit von Thiaziden, z. B. von Hydrochlorothiazid, nicht beeinflußt. Überraschend war hingegen, daß Penbutolol einen signifikanten Einfluß auf die Salidiurese von Furosemid ausübt. Die Ausscheidungswerte nach Furosemid werden durch die gleichzeitige Verabfolgung von Penbutolol (5 oder 50 mg/kg oral) in den ersten 5 Stunden herabgesetzt, jedoch erfolgt sodann ein weitgehender Ausgleich dieses Ausscheidungsdefizites in den folgenden Stunden, insbesondere der 6. bis 24. Stunde nach oraler Verabreichung beider Verbindungen. Diese Wirkung zeigt sich besonders eindrucksvoll, wenn man die Behandlung mit Furosemid allein einer Behandlung mit Furosemid und Penbutolol gegenüberstellt. Eine solche Gegenüberstellung zeigt die nachfolgende Tabelle. Es ist ersichtlich, daß bei gleichzeitiger Gabe von Penbutolol und Furosemid die Ausscheidung in den ersten 5 Stunden herabgesetzt und in den folgenden Stunden bis zur 24. Stunde erhöht ist gegenüber der Ausscheidung nach alleiniger Gabe von Furosemid. Die Endwerte beider Versuchsreihen sind im wesentlichen gleich. Ähnliche Beobachtungen wurden für Kombinationen von Penbutolol mit Piretanid gemacht.

Der überraschende Effekt einer Verzögerung der Salidiuresewirkung von Furosemid durch gleichzeitige Gabe von Penbutolol ist für die Hochdruckbehandlung sehr erwünscht, denn eine mildere und über längere Zeit anhaltende Diurese und Salurese wird für den Patienten in der Regel günstig beurteilt. Mit der erfindungsgemäßen Kombination wurde ein neues Wirkungsprinzip gefunden, das es ermöglicht gewisse high-ceiling Diuretika in einer Weise einzusetzen, daß sie beim Patienten die Wirkung eines low-ceiling-Diuretikums erzeugen.

Die zu verwendenden Dosen können individuell verschieden sein und je nach Art des zu behandelnden Zustandes variieren. Sie liegen in der Regel bei 1 bis 100 mg/Dosis für jeden Kombinationspartner bzw. der doppelten Menge eines der erfindungsgemäßen Salze. Das Verhältnis zwischen β-Blocker und Diuretikum kann beliebig variiert werden. Als besonders günstig haben sich Kombinationen zwischen dem β-Blocker und dem Diuretikum im Verhältnis 1 : 4 bis 10 : 1 erwiesen, wobei dem Verhältnis β-Blocker : Diuretikum von 2 : 1 bis 10 : 1 besondere Bedeutung zukommt. Die angegebenen Zahlen beziehen sich auf Gewichtsverhältnisse. Eine besondere Bedeutung kommt beispielsweise einem Gemisch von 20 mg Furosemid und 40 mg Penbutolol pro Dosis oder einem Gemisch von 3 bis 12 mg Piretanid und 40 mg Penbutolol pro Dosis zu.

Die Wirkstoffe können nicht nur als freie Säure bzw. Base oder als Salz des sauren Diuretikums und des basischen β-Blockers eingesetzt werden, sondern es können auch andere Salze der einzelnen Komponenten verwendet werden. Beispiele für solche Salze sind die Alkalimetallsalze der sauren Diuretika oder deren Salze mit organischen Basen wie Glucosaminen, tris-Hydroxy-äthylamin, Äthanolamin, Benzylamin oder Diäthylamin sowie die mineralsauren Salze der β-Blocker oder deren Salze mit

organischen Säuren wie Maleinsäure, Fumarsäure, Schleimsäure, Weinsäure, Glycolsäure, Brenztraubensäure, Äpfelsäure, Ameisensäure, Salicylsäure, Aminosalicylsäure. Bedingung ist, daß die salzbildende Basen- oder Säurekomponente physiologisch verträglich ist.

Die erfindungsgemäßen Mittel können gewünschtenfalls zusammen mit geeigneten pharmazeutischen Hilfsstoffen in zur oralen, intravenösen oder intramuskulären Verabreichung geeigneten galenischen Zubereitungen enthalten sein, die auch mit Mitteln zur verzögerten Wirkstoff-Freigabe kombiniert sein können. Beispiele solcher Zubereitungen sind Tabletten, Dragees, Kapseln oder Lösungen. Zur Herstellung der Zubereitungen können alle pharmazeutisch üblichen Hilfsstoffe verwendet werden.

Die Wirkstoffe können mit den Hilfsstoffen in üblicher Weise gemischt und formuliert werden. Gewünschtenfalls können feste Arzneistoffe oder deren Zwischenprodukte zur Retardierung mit einem porösen unlöslichen Film überzogen werden. Die nachfolgenden Beispiele sollen die Erfindung näher erläutern, jedoch in keiner Weise einschränken.

(Siehe Tabelle Seite 8 f.)

Tabelle

Salidiurese bei Ratten und oraler Gabe von Furosemid bzw. Furosemid und Penbutolol (1 : 2 Gew-Teile)

| Dosis *) mg/kg | Meßzeitraum | Urin (ml/kg) Furosemid | Urin (ml/kg) Furosemid und Penbutolol | $Na^+$ (mMol/kg) Furosemid | $Na^+$ (mMol/kg) Furosemid und Penbutolol |
|---|---|---|---|---|---|
| 8 | 1.-5. Std. | 17.0 | 10.7 | 0.66 | 0.35 |
| 8 | 6.-24. Std. | 17.6 | 34.5 | 1.27 | 3.39 |
| 8 | 1.-24. Std. | 34.6 | 45.2 | 1.93 | 3.74 |
| 16 | 1.-5. Std. | 39.3 | 24.0 | 3.19 | 1.37 |
| 16 | 6.-24. Std. | 13.2 | 40.9 | 1.09 | 3.39 |
| 16 | 1.-24. Std. | 52.5 | 64.9 | 4.28 | 4.76 |
| 32 | 1.-5. Std. | 62.5 | 34.3 | 6.08 | 2.88 |
| 32 | 6.-24. Std. | 13.6 | 39.7 | 1.10 | 3.97 |
| 32 | 1.-24. Std. | 76.1 | 74.0 | 7.18 | 6.85 |
| 128 | 1.-5. Std. | 83.5 | 22.8 | 8.63 | 1.86 |
| 128 | 6.-24. Std. | 27.2 | 76.5 | 2.19 | 8.08 |
| 128 | 1.-24. Std. | 110.7 | 99.3 | 10.82 | 9.94 |

*) Die Zahlen geben die Dosis für Furosemid wieder. Sofern Penbutolol ebenfalls verabreicht wurde, betrug dessen Dosis das Doppelte.

## Ausführungsbeispiele

### Beispiel 1

Kapseln, enthaltend eine Kombination aus Furosemid in Form von Pellets und Penbutolol-sulfat in Form von Tabletten :

Furosemid-pellets

| | |
|---|---|
| 1. Furosemid | 30 mg |
| 2. Pelletgrundlage | 100 mg |
| a) Saccharose | (70 mg) |
| b) Maisstärke | (30 mg) |
| 3. Polyvinylpyrrolidon K 25 | 2 mg |
| 4. Talkum | 10 mg |
| 5. Schellack | 1 mg |
| 6. Stearinsäure | 2 mg |
| | 145 mg |

In einem geeigneten Dragierkessel wird auf die Pelletgrundlage (2) eine 10 %ige Lösung von (3) in Äthanol aufgetragen und anschließend eine Mischung von (1) und (4) eingestreut.

Die Pellets, bestehend aus (1)-(4) werden mit einer 10 %igen Lösung von (5) und (6) in einem Gemisch von Äthylacetat/Äthanol lackiert.

Penbutolol-Tabletten

| | |
|---|---|
| 1. Penbutolol-sulfat | 40 mg |
| 2. Maisstärke | 14 mg |
| 3. Talkum | 3 mg |
| 4. Hochdisperses Siliciumdioxid | 2 mg |
| 5. Magnesiumstearat | 1 mg |
| | 60 mg |

Die Tablettenmischung, bestehend aus den Substanzen (1)-(5) wird granuliert und zu bikonvexen Tabletten verpreßt.

Furosemid-pellets, entsprechend einem Gehalt von 30 mg Furosemid und je 1 Penbutolol-Tablette zu 40 mg werden in Hartgelatinesteckkapseln abgefüllt.

### Beispiel 2

Tabletten, enthaltend eine Kombination aus Furosemid und Penbutolol-sulfat.

| | |
|---|---|
| 1. Furosemid | 20 mg |
| 2. Penbutolol-sulfat | 40 mg |
| 3. Lactose | 80 mg |
| 4. Maisstärke | 46 mg |
| 5. Polyvinylpyrrolidon K 25 | 5 mg |
| 6. Hochdisperses Siliciumdioxid | 4 mg |
| 7. Talkum | 4 mg |
| 8. Magnesiumstearat | 1 mg |
| | 200 mg |

Die Tablettenmischung aus den Substanzen (1) bis (8) wird granuliert und zu bikonvexen Tabletten verpreßt.

Filmtabletten

Die Tabletten werden mit einem der üblichen Filmlacke überzogen.

### Beispiel 3

Kapseln, enthaltend eine Kombination aus Piretanid in Form Pellets und Penbutolol-sulfat in Form von Tabletten.

Piretanid-pellets

| | |
|---|---|
| 1. Piretanid | 6,00 mg |
| 2. Pelletgrundlage | 140,00 mg |
| a) Saccharose | (100,00 mg) |
| b) Maisstärke | (40,00 mg) |
| 3. Polyvinylpyrrolidon K 25 | 1,00 mg |
| 4. Schellack | 2,25 mg |
| 5. Stearinsäure | 4,50 mg |
| | 153,75 mg |

In einem geeigneten Dragierkessel wird auf die Pelletgrundlage (2) eine wäßrige Suspension von (1) und (3) aufgesprüht.

Nach dem Trocknen werden die Pellets, bestehand aus (1)-(3) mit einer 10 %igen Lösung von (4) und (5) in einem Gemisch aus Äthylacetat/Äthanol lackiert.

Piretanid-pellets, entsprechend einem Gehalt von 6 mg Piretanid und je 1 Penbutololtablette zu 40 mg (s. Beispiel 1) werden in Hartgelatinekapseln abgefüllt.

Beispiel 4

Tabletten, enthaltend eine Kombination aus Piretanid und Penbutolol-sulfat.

| | |
|---|---|
| 1. Piretanid | 6 mg |
| 2. Penbutololsulfat | 40 mg |
| 3. Mikrokristalline Zellulose | 104 mg |
| 4. Maisstärke | 43 mg |
| 5. Polyvinylpyrrolidon | 6 mg |
| 6. Magnesiumstearat | 1 mg |
| | 200 mg |

Die Tablettenmischung aus den Substanzen (1) bis (6) wird granuliert und zu bikonvexen Tabletten verpreßt.

Filmtabletten

Die Tabletten werden mit einem der üblichen Filmlacke überzogen.

Beispiel 5

Herstellung eines Salzes aus Penbutolol und Furosemid

0,1 Mol (29 g) Penbutolol-Base werden in 300 ml Äthanol gelöst und unter Rühren mit einer Lösung von 0,1 Mol (33 g) Furosemid in 300 ml Aceton versetzt. Der sich nach Zugabe von Petroläther langsam abscheidende Kristallbrei wird abgesaugt, gründlich mit einem Äther/Petroläthergemisch (1 : 1) gewaschen und getrocknet.

Ausbeute : quantitativ

F : Zersetzung bei T 93-95 °C.

**Ansprüche** (für die Vertragsstaaten : BE, CH, DE, FR, GB, IT, LI, NL, SE)

1. Salz aus Furosemid oder Piretanid als Säurekomponente und Penbutolol als Basenkomponente.
2. Verfahren zur Herstellung eines Salzes gemäß Anspruch 1 dadurch gekennzeichnet, daß man Furosemid oder Piretanid und Penbutolol, vorzugsweise in Form von Lösungen, zusammenfügt.
3. Arzneimittel enthaltend mindestens zwei der Komponenten (A), (B) und (C) :

(A) Furosemid oder Piretanid
(B) Penbutolol
(C) ein Salz gemäß Anspruch 1.

4. Arzneimittel gemäß Anspruch 3 zur Behandlung von Erkrankungen des Herz- und Kreislaufsystems.

**Anspruch** (für den Vertragsstaat AT)

1. Verfahren zur Herstellung eines Salzes aus Furosemid oder Piretanid als Säurekomponente und

Penbutolol als Basenkomponente, dadurch gekennzeichnet, daß man Furosemid oder Piretanid und Penbutolol, vorzugsweise in Form von Lösungen, miteinander umsetzt.

**Claims** (for the Contracting States : (BE, CH, DE, FR, GB, IT, LI, NL, SE)

1. A salt of furosemide or piretanide as the acid component and penbutolol as the base component.
2. A process for the preparation of a salt as claimed in claim 1, which comprises bringing together furosemide or piretanide and penbutolol preferably in the form of solutions.
3. A drug containing at least two of the components (A), (B) and (C) :

(A) furosemide or piretanide,
(B) penbutolol
(C) a salt as claimed in claim 1.

4. A drug as claimed in claim 3 for the treatment of diseases of the coronary and circulatory system.

**Claim** (for the Contracting State AT)

A process for the preparation of a salt of furosemide or piretanide as the acid component and penbutolol as the base component, which comprises reacting furosemide or piretanide with penbutolol, preferably in the form of solutions.

**Revendications** (pour les Etats Contractants : BE, CH, DE, FR, GB, IT, LI, NL, SE)

1. Sel constitué par du furosémide ou du pirétanide comme constituant acide et de penbutolol comme constituant basique.
2. Procédé de préparation d'un sel selon la revendication 1, caractérisé en ce que l'on réunit du furosémide ou du pirétanide et du penbutolol, de préférence sous forme de solutions.
3. Médicament contenant au moins deux des constituants (A), (B) et (C) :

(A) Furosémide ou pirétanide
(B) Penbutolol
(C) un sel selon la revendication 1.

4. Médicament selon la revendication 3 pour le traitement des maladies du système cardio-vasculaire.

**Revendication** (pour l'Etat contractant AT)

Procédé de préparation d'un sel constitué de furosémide ou de pirétanide comme constituant acide et de penbutolol comme constituant basique, caractérisé en ce que l'on fait réagir du furosémide ou du pirétanide et du penbutolol, de préférence sous forme de solutions.